# EUROPEAN PATENT SPECIFICATION

(11) **EP 1 502 597 B1**
(45) Date of publication and mention of the grant of the patent: **10.05.2006**
(21) Application number: 04017319.7
(22) Date of filing: 22.07.2004
(51) Int. Cl.: A61K 36/48, A61K 36/41, A61P 25/22, A61P 25/24, A61P 25/00

(54) **Compositions for the treatment of anxiety and associated disorders**
Zusammensetzungen zur Behandlung von Angstzuständen und damit verbundenen Erkrankungen
Compositions pour traiter l'anxiété et de troubles associés

(30) Priority: 25.07.2003 IT MI20031534
(43) Date of publication of application: 02.02.2005
(73) Proprietor: MARFARMA HOLDING S.p.A., 20145 Milano (IT)
(72) Inventor: Heyda, Alessandro, 20145 Milano (IT)
(74) Representative: Minoja, Fabrizio

(56) References cited:
- EP-A- 0 427 529
- CA-A- 2 385 774
- DATABASE WPI Section Ch, Week 199538 Derwent Publications Ltd., London, GB; Class B04, AN 1995-290310 XP002296023 & JP 07 188039 A (ASAHI BREWERIES LTD) 25 July 1995 (1995-07-25)
- DATABASE WPI Section Ch, Week 199744 Derwent Publications Ltd., London, GB; Class B04, AN 1997-478389 XP002296024 & RU 2 076 723 C1 (LEBEDEV V V) 10 April 1997 (1997-04-10)
- DATABASE WPI Section Ch, Week 198643 Derwent Publications Ltd., London, GB; Class B04, AN 1986-281595 XP002296025 & JP 61 205218 A (AJINOMOTO KK) 11 September 1986 (1986-09-11)

## Description

This invention relates to pharmaceutical, medical, dietetic, nutritional or cosmetic compositions with an adaptogenic action based on a combination of *Griffonia simplicifolia* and *Rhodiola rosea,* which are effective in the treatment of anxiety, irritability, mood swings, nervous hunger and sleep disorders, said symptoms being present in situations such as mental or physical stress, premenstrual and menopausal syndrome and the like.

### INTRODUCTION

*Griffonia simplicifolia* and *Rhodiola rosea,* described in the literature as adaptogenic plants, namely plants able to modulate the body's resistance to stresses which alter its balance, help to maintain the psychological well-being of the individual by boosting natural resistance to mental and physical stress.

*Griffonia simplicifolia* is a plant belonging to the *Leguminosae* family, the seeds of which contain a significant quantity of 5-HTP (5-hydroxytryptophan), the precursor of serotonin, a neurotransmitter responsible for controlling the sleep, appetite and mood functions. The seeds are consequently used to prepare a dried extract for the treatment of conditions such as depression, anxiety and insomnia, and in energy products (see, for example, Zmilacher K, et al. in Neuropsychobiology 1988; 20: 28-35; van Praag H.M. et al, Schweiz Rundschau Med. (Praxis) 77, 34a; 1988, 40-46).

*Rhodiola rosea* is a plant belonging to the *Crassulaceae* family, which grows wild in Scandinavia, Siberia and North America, and is traditionally used in Siberia to alleviate feelings of tiredness. It has an adaptogenic antistress effect due to the phenylpropanoid glycosides present in the root, especially salidroside and rosavidin, which cause an increase in the production of beta-endorphins.

### DESCRIPTION OF THE INVENTION

This invention relates to pharmaceutical, medical, dietetic, nutritional or cosmetic compositions with an adaptogenic action based on a combination of *Griffonia simplicifolia* and *Rhodiola rosea* which are effective in the treatment of anxiety, irritability, mood swings, nervous hunger and sleep disorders.

In particular, it has been observed that the compositions to which this invention relates demonstrate surprisingly greater activity than would be expected in view of the activities of the individual active substances, thus demonstrating the presence of a synergic action between the two components which leads to a particularly strong adaptogenic effect.

The compositions according to the invention are useful in the treatment of conditions of anxiety, irritability, mood swings, nervous hunger and sleep disorders which are associated, for example, with situations of mental or physical stress, premenstrual or menopausal syndrome. One of the characteristic features of both premenstrual and menopausal syndrome is nervousness, emotional instability, irritability, aggressiveness, anxiety, depression and mood changes. The same disorders are present in persons subject to conditions of great stress. Moreover, in the case of both premenstrual syndrome and general stress, the psychological fragility of the individual can also be manifested by episodes of nervous hunger.

According to a preferred aspect of the invention, *Griffonia simplicifolia* is used in the form of an extract of the seeds contained in the pods of the plant, titrated in 5-hydroxytryptophan and containing said active constituent at the concentration of 20%.

According to a further preferred aspect of the invention, *Rhodiola rosea* is used in the form of an extract standardised to contain 3% salidroside.

The compositions according to the invention will preferably contain between 25 and 300 mg of *Griffonia simplicifolia* extract and between 50 and 150 mg of *Rhodiola rosea* extract per administration unit.

The compositions according to the invention will preferably be administered in such quantities as to provide a daily intake of 50 - 750 mg of *Griffonia simplicifolia* extract titrated in 5-hydroxytryptophan and containing said active constituent at the concentration of 20%; and 100 - 200 mg of *Rhodiola rosea* extract standardised to contain 3% salidroside.

The compositions of the invention may also contain other active ingredients appropriate for the treatment of other conditions.

For example, for the treatment of conditions of anxiety, irritability, mood swings, nervous hunger and sleep disorders associated with situations of biological/physical (oxidative) or psychological stress, particularly in elderly persons, the compositions to which this invention relates could also contain compounds with an antioxidant effect chosen from the group of selenium, vitamins A, C and E, and lipoic acid.

For the treatment of conditions of anxiety, irritability, mood swings, nervous hunger and sleep disorders associated with premenstrual or menopausal syndrome, the compositions to which this invention relates could also contain compounds such as soya isoflavones, *Cimicifuga racemosa* (black cohosh) extract, *Vitex agnus castus* (chaste tree) extract, red clover extract or borage oil. Soya isoflavones are phyto-oestrogens which have long been studied for their oestrogenic and antioxidant effect and used in the treatment of menopausal syndrome. *Cimicifuga racemosa* is a plant of the *Ranunculaceae* family, the roots and rhizome of which contain the natural oestrogen formononetin together with triterpene glycosides, cimicifugin and other substances. The extract, usually titrated to contain triterpene glycosides at values of between 0.5 and 30%, has proved effective at relatively low doses, usually not exceeding 40 mg a day, in treating the neurovegetative disorders associated with the menopause. Chaste-tree is a plant belonging to the *Verbenaceae* family, the fruit of which contains a mixture of flavonoids, iridoids and hormone-like compounds which perform an action similar to that of corpus luteum; it is consequently used to treat premenstrual syndrome. Finally, the oil extracted from the seeds of *Borrago officinalis* (borage) has a high gamma-linolenic acid content, whose favourable effect on premenstrual syndrome is well known.

The compositions of the invention can be formulated in a way suited to oral, parenteral, transdermal or transmucosal administration in accordance with conventional techniques and excipients, as described in "Remington's Pharmaceutical Science Handbook", 17th Ed., Mack Pub., N.Y., U.S.A.

Examples of these formulations are liquids and suspensions, injectable forms, sprays, inhalers, gel, emulsions, tablets, capsules, suppositories, transdermal patches, granulates and powders. Gastro-protected and/or modified-release formulations are preferred for oral administration, especially modified-release tablets which release the active constituents 5-8 hours after administration, obtainable by means of polymers or copolymers conventionally used for the purpose, such as acrylic or methacrylic acid copolymers (Eudragit® ) or cellulose derivatives. In the case of transdermal applications, an absorption promoter such as liposomes or a cross-linked polymer which traps the functional substances could also be used.

The following examples illustrate the invention in greater detail.

### Example 1: tablets

| **COMPONENT** | **mg/tablet** | **RDA** |
|---|---|---|
| *Griffonia simplicifolia* dried extract (5-http) | 50 | |
| *Rhodiola rosea* | 125 | |
| Selenium | 82 mcg | * |
| Vitamin A | 0.45 | 56 |
| Vitamin C | 180 | 300 |
| Vitamin E | 30 | 300 |
| Lipoic acid | 50 | |
| Magnesium oxide | 422.745 | 37.41 |
| Filling agent: calcium phosphate | 267.804 | 23.70 |
| Filling agent: microcrystalline cellulose | 172.482 | 15.26 |
| Anti-caking agent: silicon dioxide | 32.620 | 2.89 |
| Coating agent: hydroxypropyl methylcellulose | 13.800 | 1.22 |
| Anti-caking agent: magnesium stearate (vegetable) | 11.000 | 0.97 |
| Zinc oxide | 9.335 | 0.83 |
| Coating agent: shellac | 7.000 | 0.62 |
| Colouring: titanium dioxide | 3.450 | 0.31 |
| Anti-caking agent: E427a | 2.300 | 0.20 |
| Colouring: cochineal extract | 0.008 | 0.00 |

| | | |
|---|---|---|
| RDA = Recommended Dietary Allowance | | |

### Example 2: 920 mg tablets

| **COMPONENT** | **mg/tablet** | **%** |
|---|---|---|
| *Griffonia simplicifolia (seeds)* dried extract (27.2%) | 250.000 | 27.17 |
| *Rhodiola* extract titrated to contain 3% salidroside | 125.00 | |
| Soya extract titrated to contain 15% isoflavones and 26.5% saponins (43.5%) | 400.000 | 43.48 |
| *Cimifuga racemosa* (rhizome) dried extract titrated to contain 2.5% triterpene glycosides calculated as 27-deoxyactein (4.35%) | 40.000 | 4.35 |
| Coating agent: hydroxypropyl methylcellulose | 140.000 | 15.22 |
| Filling agent: microcrystalline cellulose | 65.800 | 7.15 |
| Anti-caking agent: silicon dioxide | 15.000 | 1.63 |
| Anti-caking agent: magnesium stearate (vegetable) | 9.200 | 1.00 |
| **TOTAL** | 1.045 | |

### Example 3: 1.13 g tablets

| C**OMPONENT** | **UNIT DOSE** | **%** |
|---|---|---|
| *Griffonia simplicifolia (seeds)* dried extract (5.75%) | 65.000 | 5.75 |
| *Rhodiola* extract titrated to contain 3% salidroside | 125.00 | |
| *Vitex Agnus castus*, fruit, dried extract, titrated to contain 0.01% vitexin (4.42%) | 50.000 | 4.42 |
| Borage oil (*Borrago officinalis*) (1.77%) | 20.000 | 1.77 |
| Vitamin E acetate | 20.000 | 1.77 |
| Vitamin B6 hydrochloride | 12.228 | 1.08 |
| Vitamin B1 nitrate | 9.421 | 0.83 |
| Vitamin B2 | 8.727 | 0.77 |
| Vitamin D | 2.080 | 0.18 |
| Magnesium oxide | 422.745 | 37.41 |
| Filling agent: calcium phosphate | 267.804 | 23.70 |
| Filling agent: microcrystalline cellulose | 172.482 | 15.26 |
| Anti-caking agent: silicon dioxide | 32.620 | 2.89 |
| Coating agent: hydroxypropyl methylcellulose | 13.800 | 1.22 |
| Anti-caking agent: magnesium stearate (vegetable) | 11.000 | 0.97 |
| Zinc oxide | 9.335 | 0.83 |
| Coating agent: shellac | 7.000 | 0.62 |
| Colouring: titanium dioxide | 3.450 | 0.31 |
| Anti-caking agent: E427a | 2.300 | 0.20 |
| Colouring: cochineal extract | 0.008 | 0.00 |
| **TOTAL** | 1.155 | |

## Claims

1. Pharmaceutical, medical, dietetic, nutritional or cosmetic compositions with an adaptogenic action based on a combination of *Griffonia simplicifolia* and *Rhodiola rosea*, which are effective in the treatment of anxiety, irritability, mood swings, nervous hunger and sleep disorders associated with premenstrual syndrome, menopausal syndrome, and conditions of mental or physical stress.

2. Compositions as claimed in claim 1, containing *Griffonia simplicifolia* in amounts between 25 and 300 mg and *Rhodiola rosea* in amounts between 50 and 150 mg.

3. Compositions as claimed in the preceding claims, further containing antioxidants selected from the group of selenium, vitamins A, C and E, and lipoic acid.

4. Compositions as claimed in the preceding claims, further containing soya isoflavones.

5. Compositions as claimed in the preceding claims, further containing *Vitex agnus castus* extract.

6. Compositions as claimed in the preceding claims, further containing *Cimicifuga racemosa* extract.

7. Compositions as claimed in the preceding claims, further containing red clover extract.

8. Compositions as claimed in the preceding claims, further containing borage oil.

9. Compositions as claimed in the preceding claims, suitable for oral administration.

10. Compositions as claimed in any one of the preceding claims, in the form of tablets.

## Patentansprüche

1. Pharmazeutische, medizinische, diätetische, alimentäre oder kosmetische Zusammensetzungen mit adaptogener Wirkung basierend auf einer Kombination von *Griffonia simplicifolia* und *Rhodiola rosea*, welche wirksam sind bei der Behandlung von Angst, Reizbarkeit, Stimmungsschwankungen, nervösem Hunger und Schlafstörungen im Zusammenhang mit prämenstruellem Syndrom, menopausalem Syndrom und Zuständen von psychischem oder physischem Stress.

2. Zusammensetzungen nach Anspruch 1, enthaltend *Griffonia simplicifolia* in Mengen zwischen 25 und 300 mg und *Rhodiola rosea* in Mengen zwischen 50 und 150 mg.

3. Zusammensetzungen nach einem der vorstehenden Ansprüche, weiter enthaltend Antioxidantien ausgewählt aus der Gruppe von Selen, Vitaminen A, C und E und Liponsäure.

4. Zusammensetzungen nach einem der vorstehenden Ansprüche, weiter enthaltend Sojaisoflavone.

5. Zusammensetzungen nach einem der vorstehenden Ansprüche, weiter enthaltend *Vitex agnus castus* Extrakt.

6. Zusammensetzungen nach einem der vorstehenden Ansprüche, weiter enthaltend *Cimicifuga racemosa* Extrakt.

7. Zusammensetzungen nach einem der vorstehenden Ansprüche, weiter enthaltend Rotkleeextrakt.

8. Zusammensetzungen nach einem der vorstehenden Ansprüche, weiter enthaltend Borretschöl.

9. Zusammensetzungen nach einem der vorstehenden Ansprüche, geeignet für die orale Verabreichung.

10. Zusammensetzungen nach einem der vorstehenden Ansprüche in Form von Tabletten.

## Revendications

1. Compositions pharmaceutiques, médicales, diététiques, nutritionnelles ou cosmétiques ayant une action adaptogène basées sur une combinaison de *Griffonia simplicifolia* et de *Rhodiola rosea*, qui sont efficaces dans le traitement de l'anxiété, l'irritabilité, les sautes d'humeur, la boulimie nerveuse et les désordres du sommeil associés au syndrome prémenstruel, au syndrome de la ménopause, et des états de stress mental ou physique.

2. Compositions selon la revendication 1, contenant *Griffonia simplicifolia* en des quantités entre 25 et 300 mg et *Rhodiola rosea* en des quantités entre 50 et 100 mg.

3. Compositions telles que revendiquées dans les revendications précédentes, contenant en outre des anti-oxydants choisis dans le groupe du Sélénium, des vitamines A, C et E et de l'acide lipoïque.

4. Compositions telles que revendiquées dans les revendications précédentes, contenant en outre des isoflavones de soja.

5. Compositions telles que revendiquées dans les revendications précédentes, contenant en outre de l'extrait de *Vitex agnus castus.*

6. Compositions telles que revendiquées dans les revendications précédentes, contenant en outre de l'extrait de *Cimicifuga racemosa.*

7. Compositions telles que revendiquées dans les revendications précédentes, contenant en outre de l'extrait de trèfle violet.

8. Compositions telles que revendiquées dans les revendications précédentes, contenant en outre de l'huile de bourrache.

9. Compositions telles que revendiquées dans les revendications précédentes, appropriées pour une administration orale.

10. Compositions telles que revendiquées dans l'une quelconque des revendications précédentes, sous forme de comprimés.
